# EUROPEAN PATENT SPECIFICATION

(11) **EP 2 913 850 B1**
(45) Date of publication and mention of the grant of the patent: **12.09.2018**
(21) Application number: 13849748.2
(22) Date of filing: 04.10.2013
(51) Int. Cl.: H01L 27/14, H04N 5/335

(54) **IMAGE PICKUP APPARATUS AND ENDOSCOPE**
BILDAUFNAHMEVORRICHTUNG UND ENDOSKOP
DISPOSITIF D'IMAGERIE ET ENDOSCOPE

(30) Priority: 23.10.2012 JP 2012233984; 12.11.2012 JP 2012248676
(43) Date of publication of application: 02.09.2015
(60) Divisional application: 18185215.3
(73) Proprietor: Olympus Corporation, Tokyo 192-8507 (JP)
(72) Inventor: YOSHIDA Kazuhiro, Shibuya-ku, Tokyo 151-0072 (JP); NAKAYAMA Takashi, Shibuya-ku, Tokyo 151-0072 (JP)
(74) Representative: Winter, Brandl, Fürniss, Hübner, Röss, Kaiser, Polte - Partnerschaft mbB
(86) International application number: PCT/JP2013/077113
(87) International publication number: WO 2014/065099

(56) References cited:
- JP-A- 2001 326 484
- JP-A- 2001 326 484
- JP-A- 2008 177 402
- JP-A- 2008 288 273
- JP-A- 2010 199 352
- JP-A- 2011 060 933
- JP-A- 2011 177 262
- JP-A- 2012 064 883
- US-A1- 2009 027 491
- US-A1- 2011 199 473
- US-A1- 2011 249 106
- US-B1- 6 313 456

## Description

### Technical Field

The present invention relates to an image pickup apparatus having an image pickup device chip, an endoscope provided with the image pickup apparatus.

### Background Art

An image pickup apparatus having an image pickup device chip is used, for example, by being disposed at a distal end portion of an endoscope. Regarding the distal end portion of the endoscope, there has been a considerable problem of thinning a diameter thereof in order to reduce pain on an examinee.

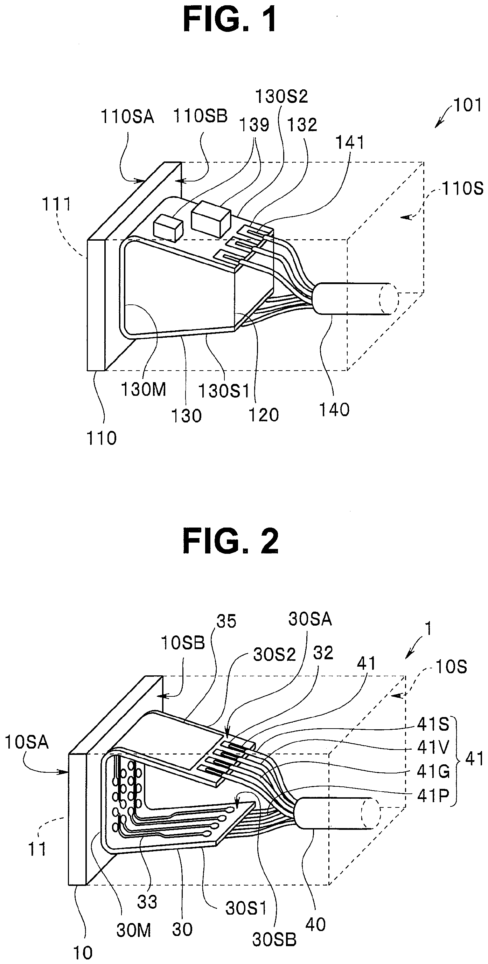

As shown in FIG. 1, in Japanese Patent Laid-Open Publication No. 2011-217887, there is described an image pickup apparatus 101 having an image pickup device chip 110, a block 120 which is a heat radiation member, a wiring board 130 on which electrical parts 139 are mounted, and a signal cable 140. The image pickup device chip 110 has in image pickup unit 111 on an obverse surface 110SA and a plurality of junction terminals (not shown) on a reverse surface 110SB. The junction terminals are joined to junction electrodes (not shown) of the wiring board 130. The junction electrodes are connected with terminal electrodes 132, which are joined to lead wires 141 of the signal cable 140, through wirings (not shown).

The wiring board 130 is comprised of a central portion 130M which is joined to the image pickup device chip 110 and extending portions 130S1 and 130S2 which extend from the central portion 130M on respective sides thereof. The extending portions 130S1 and 130S2 are bent inward. Therefore, the wiring board 130 is housed in an interior 110S of an extended space of a projected plane (within the projected plane) of the image pickup device chip 110. In the image pickup apparatus 101, heat generated by the image pickup device chip 110 is transmitted to the block 120 through the wiring board 130.

However, in the image pickup apparatus 101, there has been a fear that a temperature of the image pickup device chip 110 rises to deteriorate images if a heat transmitting function of the wiring board 130, which is located between the image pickup device chip 110 and the block 120, is insufficient.

It is noted that in Japanese Patent Laid-Open Publication No. 2010-199352, there is described a circuit board provided with a signal pattern, a power supply pattern and a heat radiation pattern. The heat radiation pattern is positioned at a region other than regions of the signal pattern and the power supply pattern and is electrically connected with the power supply pattern.

However, in the above circuit board, there is a fear that a heat amount that can be radiated from the heat radiation pattern is insufficient in a case where the circuit board is used in an image pickup apparatus which generates a large amount of heat, etc., and it has not been easy to apply the circuit board, as it is, to an ultra-small image pickup apparatus that is disposed at a distal end portion of an endoscope which has importance in thinning a diameter thereof.
From US 2011/249106 A1 an Image pickup apparatus in accordance with the preamble of claim 1 is known. A similar apparatus is known from JP 2001-326484 A.

### Summary of Invention

### Technical Problem

An object of the present invention is to provide an image pickup apparatus capable of being disposed in a narrow space, having an excellent heat radiation characteristic and allowing incorporation of various types of image pickup device chips, an endoscope provided with the image pickup apparatus.

### Means for Solving the Problem

An image pickup apparatus according to an aspect of the present invention includes: an image pickup device chip that has an image pickup unit on an obverse surface and junction terminals on a reverse surface, the junction terminals being connected with the image pickup unit via through wirings; a signal cable having lead wires connected with the image pickup unit; and a wiring board that is constituted by a central portion and a plurality of extending portions extending from the central portion, and includes junction electrodes formed at the central portion and joined to the junction terminals, terminal electrodes formed at the extending portions and connected with the lead wires, wirings that connect the junction electrodes and the terminal electrodes, and a heat transmission pattern formed in a region where the junction electrodes, the terminal electrodes and the wirings are not formed, the extending portions being bent and thereby the wiring board being arranged within a projected plane of the image pickup device chip. The image pickup device chip has a dummy junction terminal that has the same shape as the junction terminal and is not connected with the image pickup unit, on a reverse surface, and the wiring board has a dummy junction electrode that has the same shape as the junction electrode and is connected with the dummy junction terminal.

An endoscope according to another aspect of the present invention includes: an insertion portion having a distal end portion at which an image pickup apparatus is disposed, the image pickup apparatus including: an image pickup device chip that has an image pickup unit on an obverse surface and junction terminals on a reverse surface, the junction terminals being connected with the image pickup unit via through wirings; a signal cable having lead wires connected with the image pickup unit; and a wiring board that is constituted by a central portion and a plurality of extending portions extending from the central portion, and includes junction electrodes formed at the central portion and joined to the junction terminals, terminal electrodes formed at the extending portions and connected with the lead wires, wirings that connect the junction electrodes and the terminal electrodes, and a heat transmission pattern formed in a region where the junction electrodes, the terminal electrodes and the wirings are not formed, the extending portions being bent and thereby the wiring board being arranged within a projected plane of the image pickup device chip; an operation portion disposed on a proximal end side of the insertion portion; and a universal cord extending from the operation portion.

### Advantageous Effects of Invention

According to embodiments of the present invention, an image pickup apparatus capable of being disposed in a narrow space, having an excellent heat radiation characteristic and allowing incorporation of various types of image pickup device chips, and an endoscope provided with the image pickup apparatus can be provided.

### Brief Description of the Drawings

FIG. 1 is a perspective view of a conventional image pickup apparatus;
FIG. 2 is a perspective view of an image pickup apparatus according to a first embodiment;
FIG. 3 is a cross sectional view of the image pickup apparatus according to the first embodiment;
FIG. 4 is a plan view of a reverse surface of an image pickup device chip of the image pickup apparatus according to the first embodiment;
FIG. 5 is an exploded sectional view for explaining a manufacturing method of the image pickup apparatus according to the first embodiment;
FIG. 6 is a plan view of a first primary surface of a wiring board of the image pickup apparatus according to the first embodiment;
FIG. 7 is a plan view of a second primary surface of the wiring board of the image pickup apparatus according to the first embodiment;
FIG. 8 is a plan view of a first primary surface of a wiring board of an image pickup apparatus according to a second embodiment;
FIG. 9 is a plan view of a second primary surface of the wiring board of the image pickup apparatus according to the second embodiment;
FIG. 10 is a plan view of a first primary surface of a wiring board of an image pickup apparatus according to a third embodiment;
FIG. 11 is a plan view of a second primary surface of the wiring board of the image pickup apparatus according to the third embodiment;
FIG. 12 is a plan view of a first primary surface of a wiring board of an image pickup apparatus according to a fourth embodiment;
FIG. 13 is a plan view of a second primary surface of the wiring board of the image pickup apparatus according to the fourth embodiment;
FIG. 14 is an exploded view of the image pickup apparatus according to the fourth embodiment;
FIG. 15 is a cross sectional view of an image pickup apparatus according to a fifth embodiment;
FIG. 16 is a cross sectional view of an image pickup apparatus according to a sixth embodiment; and
FIG. 17 is an external view of an endoscope according to a seventh embodiment.

### Best Mode for Carrying Out the Invention

### <First Embodiment>

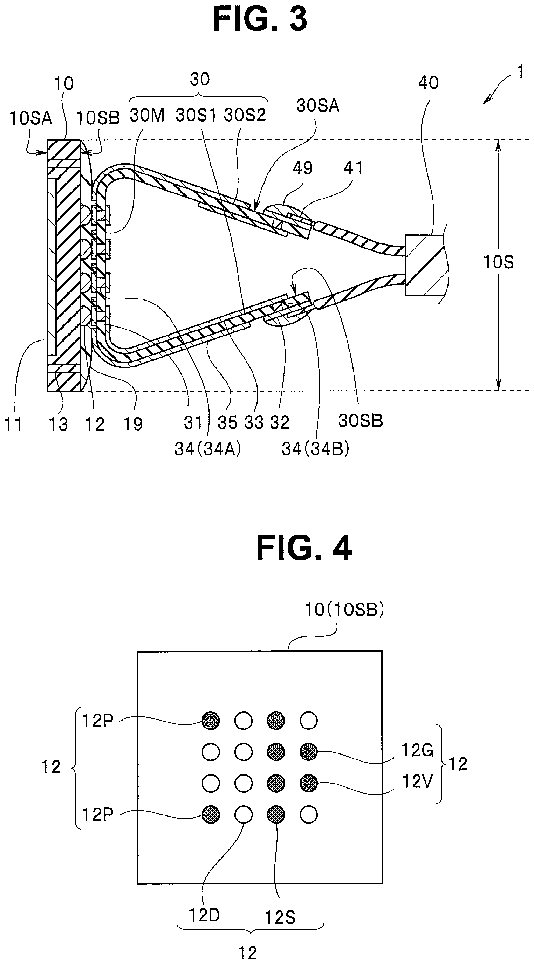

An image pickup apparatus 1 according to a first embodiment, as shown in FIG. 2 and FIG. 3, is similar to the conventional image pickup apparatus 101 already described. That is, the image pickup apparatus 1 is provided with an image pickup device chip 10, a wiring board 30, a signal cable (hereinafter also simply referred to as "cable") 40.

The wiring board 30 is constituted by a central portion 30M which is joined to the image pickup device chip 10 on a side of a first primary surface 30SA, and extending portions 30S1, 30S2 which are provided to extend from the central portion 30M on respective sides thereof. The extending portions 20S1, 30S2 are bent toward a second primary surface 30SB. Therefore, the wiring board 30 is located to be enclosed in an interior 10S of an extended space of a projected plane (within the projected plane) of the image pickup device chip 10.

On an obverse surface 10SA of the image pickup device chip 10 which is comprised of a semiconductor, there is formed an image pickup unit 11 of a CMOS device, for example, which is a solid-state image pickup device. The image pickup device chip 10 has a plurality of junction terminals 12, which are connected with the image pickup unit 11 on the obverse surface 10SA via respective through wirings 13, on a reverse surface 10SB. It is noted that wirings for connecting the image pickup unit 11 and the through wirings 13 are formed on the obverse surface 10SA but these wirings are not shown.

The image pickup device chip 10 are manufactured, for example, using a known semiconductor process, by forming a number of image pickup units 11, through wirings 13, etc. on a silicone substrate, and then cutting the substrate. Therefore, the image pickup device chip 10 has an approximately rectangular shape in a plan view. It is preferable that dimensions of the image pickup device chip 10 in the plan view, in other words, an area of the primary planes is small in order to reduce a diameter of the image pickup apparatus 1.

Here, as shown in FIG. 3 and FIG. 4, the image pickup device chip 10 of the image pickup apparatus 1 has sixteen junction terminals 12 having the same shape in appearance. However, eight terminals out of the above terminals are dummy junction terminals 12D which are not connected with the image pickup unit 11. In FIG. 4, the dummy junction terminals 12D are indicated by white circles. That is, the eight terminals are the dummy junction terminals 12D, excluding eight terminals of a junction terminal 12V for supplying power to the image pickup unit 11, two junction terminals 12P for supplying a pulse signal to the image pickup unit 11, four junction terminals 12S for transmitting and receiving signals to and from the image pickup unit 11, and a junction terminal 12G for causing the image pickup unit 11 to have the ground potential.

The junction terminal 12V is connected with a lead wire 41V of the cable 40 which supplies the power, the junction terminals 12P are connected with lead wires 41P which supply the pulse signal, the junction terminals 12S are connected with lead wires 41S which transmit and receive the signals, and the junction terminal 12G is connected with a lead wire 41G of the ground potential. (Hereinafter, the lead wires 41V, 41P, 41S and 41G are also referred to simply as "lead wires 41".)

The number of junction terminals 12 and the number of dummy junction terminals 12D and arrangements thereof are selected in accordance with specifications of the image pickup apparatus. Further, functions and the like of the junction terminals 12 are not limited to the above configuration. For example, there may be provided one junction terminal 12P or a plurality of junction terminals 12G. (Hereinafter, the dummy junction terminals 12D, and the junction terminals 12V, 12P, 12S and 12G are also referred to simply as "junction terminals 12").

Terminal electrodes 32 of the wiring board 30 and lead wires 41 of the cable 40 are joined by solder 49, for example. It is noted that the lead wire 41G of the ground potential may be a shield wire covering circumferences of the other lead wires. Further, each of the lead wires may have a shield wire. Particularly, it is preferable that the lead wires 41P and the lead wires 41S, which tend to cause a noise or be influenced by a noise, are shield lines. It is not necessary that the cable 40 is arranged in the interior 10S of the extended space of the projected plane (hereinafter referred to as "within the projected plane") over the entire length of the cable, and it is sufficient that at least a joining portion to the wiring board 30 is arranged in the interior 10S of the projecting plane.

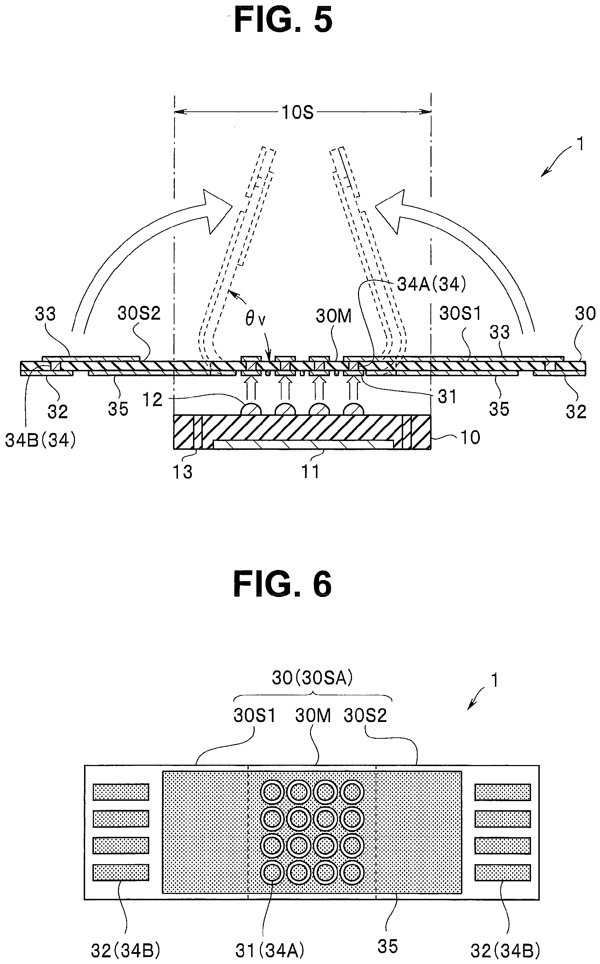

As shown in FIG. 5 and FIG. 6, the wiring board 30 is arranged in the interior 10S of the projected plane of the image pickup device chip 10 by bending the extending portions 30S1 and 30S2 with the first primary surface 30SA located outside (with the second primary surface 30SB located inside). A bending angle θv is preferably set to an angle of 90-50 degrees and particularly preferably set to an angle of 75-55 degrees, and is for example an angle of 65 degrees. If the bending angle is set within the above range, the joining portion of the wiring board 30 and the cable 40 can be arranged in the interior 10S of the projected plane of the image pickup device chip 10. It is noted that there is a case where an angle of 90 degrees is most preferable as the bending angle θv, as described later.

Since the wiring board 30 is one flexible wiring board, boundaries of the central portion 30M and the extending portions 30S1, 30S2 are not clearly defined. Besides, it may be configured that at least bent portions of the wiring board are flexible and the wiring board is a rigid flexible wiring board in which part or all of the central portion 30M and extending portions 30S1 and 30S2 is constituted by a rigid substrate.

The wiring board 30 has junction electrodes 31, which are joined to the junction terminals 12 of the image pickup device chip 10, on the first primary surface 30SA in the central portion 30M. The wiring board 30 has sixteen junction electrodes 31 in appearance but eight electrodes of these are dummy junction electrodes 31D which are not connected with the image pickup unit 11.

The eight dummy junction electrodes 31D are respectively joined to the dummy junction terminals 12D. Therefore, the image pickup apparatus 1 is configured such that the image pickup device chip 10 and the wiring board 30 are easily jointed in parallel and joining strength is large. Further, heat generated by the image pickup device chip 10 can be transmitted to the wiring board 30 more efficiently.

Besides, as shown in FIG. 3, it is preferable that a space between the image pickup device chip 10 and the wiring board 30 is sealed by a sealing resin 19. A gold bump, a solder ball, an ACP (anisotropic conductive plastic), an ACF (anisotropic conductive film) or the like is used for joining the junction terminals 12 and the junction electrodes 31.

The junction electrodes 31 on the first primary surface 30SA are connected with wirings 33 on the second primary surface 30SB via through wirings 34A, and the wirings 33 are connected with terminal electrodes 32 on the first primary surface 30SA of the extending portions 30S1, 30S2 via through wirings 34B. Hereinafter, the through wirings 34A and 34B are referred to as through wirings 34. It is noted that the dummy junction electrodes 31D may have connection portions on the second primary surface 30SB via through wirings. However, the dummy junction electrodes 31D are not connected with any electrode other than the ground potential wire (lead wire 41G).

Since the wiring board 30 is a both-side wiring board, the junction electrodes 31 and the terminal electrodes 32 are connected with one another through the wires 33 and the through wires 34A, 34B, but in a case of a one-side wiring board, through wirings for connection of the junction electrodes 31 and the terminal electrodes 32 are unnecessary. In contrast, a multi-layer wiring board having three wiring layers or more may be used. Further, an electronic part 13may be implemented in the middle of the wiring 33. As the electronic part, a necessary part is selected from among a chip condenser, a chip resister, a signal processing IC, a driver IC, a power supply IC, a diode, a coil, a lead switch, etc.

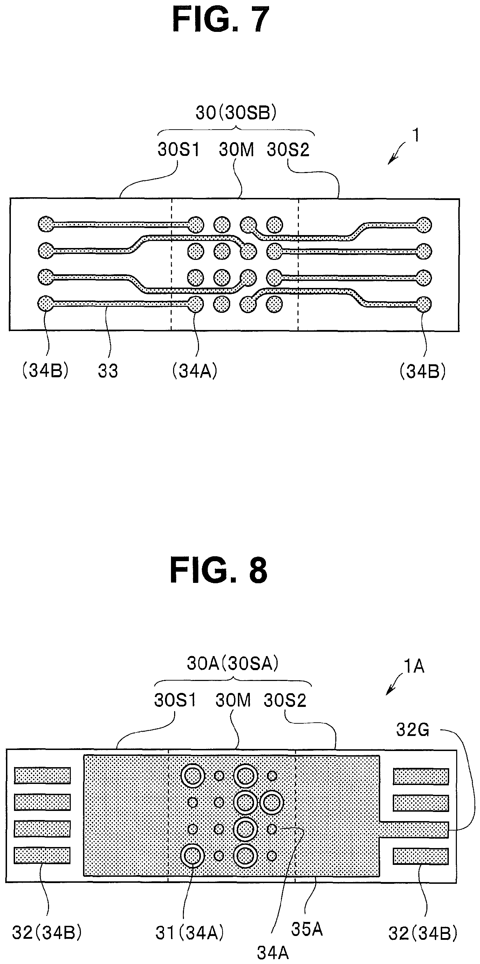

Further, as shown in FIG. 6 and FIG. 7, the wiring board 30 has a heat transmission pattern 35, which is formed in a region where the junction electrodes 31 and the terminal electrodes 32 are not formed, on the first primary surface 30SA. In the wiring board 30 exemplified in FIG. 6, the heat transmission pattern 35 is formed from the central portion 30M to the extending portions 30S1, 30S2, to surround the junction electrodes 31. The heat transmission pattern 35 transmits heat generated by the image pickup unit 11 to a proximal end portion side.

As shown in FIG. 7, no heat transmission pattern is formed on the second primary surface 30SB, but a second heat transmission pattern may be formed in a region where the wirings 33 are not formed.

The junction electrodes 31, the terminal electrodes 32 and the heat transmission pattern 35 are formed, for example, by pattern-etching a conductive film which is formed on entirety of the first primary surface 30SA. That is, the conductive film is removed by etching in a frame shape so that the junction electrodes 31 and the heat transmission pattern 35 are not electrically connected. As a matter of course, the heat transmission pattern 35, etc. may be formed by a plating method. Further, the heat transmission pattern 35 may be formed into a film shape to be thicker than the junction electrodes 31, the terminal electrodes 32, etc.

It is preferable that an area of the heat transmission pattern 35 is large and it is particularly preferable that the area is not less than 50% and not more than 90% of an area of the first primary surface 30SA. If the area is not less than the above lower limit, an effect of improving a heat radiation characteristic is remarkable and if the area is not more than the above upper limit, a necessary area for the junction electrodes 31 and the terminal electrodes 32 can be secured.

Besides, it may be configured such that the terminal electrodes 32 and the wirings 33 are formed in at least one of the extending portions 30S1 and 30S2, and the heat transmission pattern 35 is formed in at least one of the extending portions 30S1 and 30S2.

It is easy to reduce external dimensions of the image pickup apparatus 1 since the wiring board 30, etc. can be arranged in a predetermined narrow space. Therefore, the image pickup apparatus 1 is capable of being disposed stably in a narrow space. Further, the heat generated by the image pickup device chip 10 is transmitted to the proximal end side through the heat transmission pattern 35. Thus, the image pickup apparatus 1 is excellent in the heat radiation characteristic.

In addition, since the dummy junction terminals 12D of the image pickup device chip 10 and the dummy junction electrodes 31D of the wiring board 30 are joined to one another, the image pickup apparatus 1 is further excellent in the heat radiation characteristic.

### <Second Embodiment>

Next, an image pickup apparatus 1A according to a second embodiment will be described. The image pickup apparatus 1A is similar to the image pickup apparatus 1 and therefore the same reference signs are assigned to the same elements and the description thereof is omitted.

As shown in FIG. 8, in a wiring board 30A of the image pickup apparatus 1A, a terminal electrode 32G, which is connected with the lead wire 41G of the ground potential of the cable 40, is integrated with a heat transmission pattern 35A. Further, the dummy junction electrodes 31D are also integrated with the heat transmission pattern 35A. That is, the dummy junction electrodes 31D and the heat transmission pattern 35A are connected with each other. It is noted that a first primary surface 30SA of the wiring board 30A is covered with a resin layer (not shown) except for joining regions with the junction terminals 12 of the image pickup device chip 10.

The image pickup apparatus 1A has the effects of the image pickup apparatus 1 and further since the heat transmission pattern 35A having a wide area is at the ground potential, noise radiation caused by transmission and reception of signals is prevented and the apparatus is less influenced by a noise from outside. Further, the image pickup apparatus 1A has a higher heat radiation effect than the image pickup apparatus 1 since the dummy joining electrodes 31D are connected with the heat transmission pattern 35A.

### <Third Embodiment>

Next, an image pickup apparatus 1B according to a third embodiment will be described. The image pickup apparatus 1B is similar to the image pickup apparatuses 1 and 1A, and therefore the same reference signs are assigned to the same elements and the description thereof is omitted.

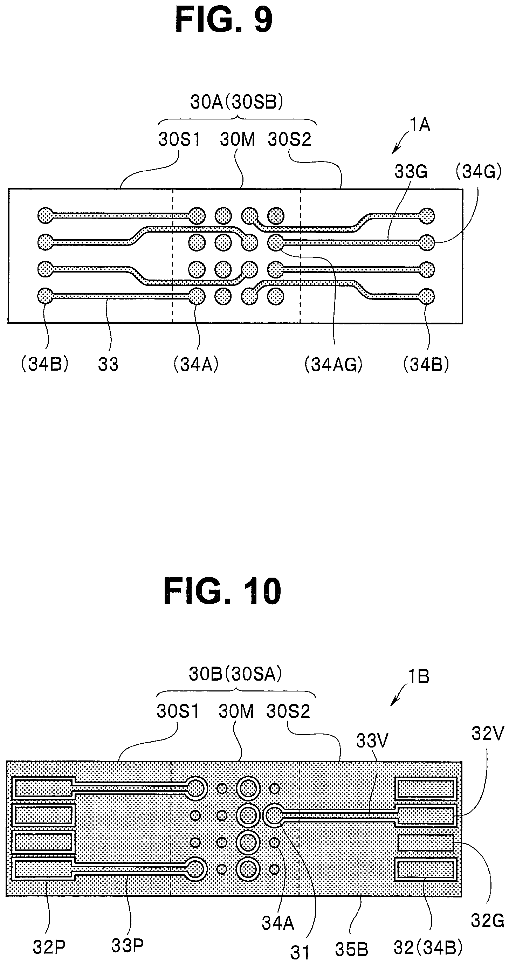

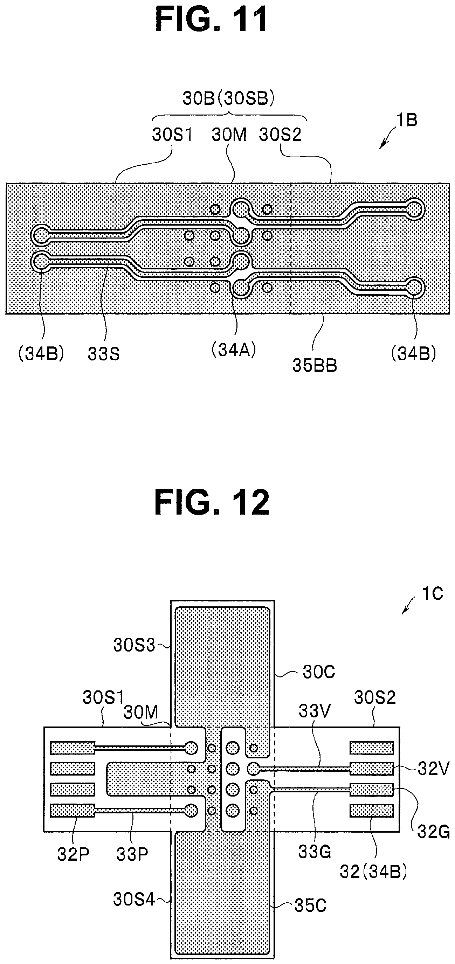

As shown in FIG. 10 and FIG. 11, a wiring board 30B of the image pickup apparatus 1B includes, on the first primary surface 30SA, a power supply wiring 33V for supplying power to the image pickup unit 11, terminal electrodes 32P and pulse wirings 33P which are joined to the lead wires 41P for supplying a clock signal as the pulse signal and a heat transmission pattern 35B, and includes on the second primary surface 30SB, terminal electrodes 32S and signal wirings 33S which are joined to the lead wires 41S for transmitting and receiving signals to and from the image pickup apparatus 11.

The image pickup apparatus 1B has the effects of the image pickup apparatuses 1 and 1A, and further since the pulse wirings 33P and the signal wirings 33S are formed on different primary surfaces of the wiring board 30B, deterioration of a received image caused by interference of the signals is prevented.

Besides, as long as the pulse wirings 33P and the signal wirings 33S are respectively formed on the different primary surfaces, the number of wirings, arrangements thereof, etc. are not limited to the above configuration and are selected according to specifications of the image pickup apparatus.

Further, since the wiring board 30B has a second heat transmission pattern 35BB, which is at the ground potential, further on the second primary surface 30SB, the image pickup apparatus 1B has a higher heat radiation effect and a higher shield effect than the image pickup apparatuses 1 and 1A.

### <Fourth Embodiment>

Next, an image pickup apparatus 1C according to a fourth embodiment will be described. The image pickup apparatus 1C is similar to the image pickup apparatuses 1, 1A and 1B, and therefore the same reference signs are assigned to the same elements and the description thereof is omitted.

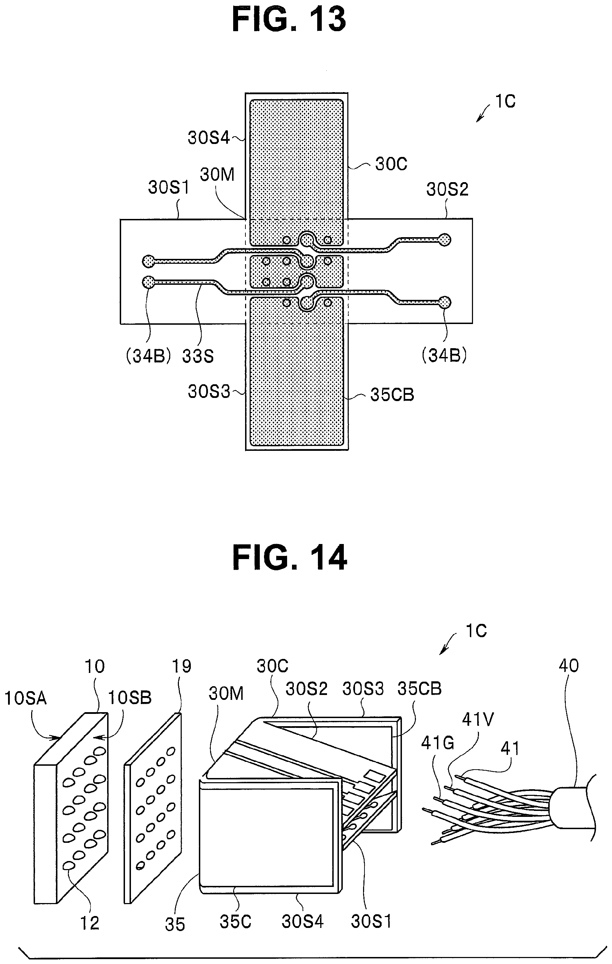

As shown in FIG. 12 and FIG. 13, a wiring board 30C of the image pickup apparatus 1C includes a central portion 30M having a substantially squire shape, and four extending portions 30S1-30S4 which have substantially rectangular shapes and extend from the central portion 30M in directions orthogonal with each other.

As shown in FIG. 14, the four extending portions 30S1-30S4 are all bent inward and therefore the wiring board 30C is arranged in the interior 10S of the projected surface of the image pickup device chip 10.

It is noted that a bending angle θv of the extending portions 30S1 and 30S2 is set to an angle of 75-55 degrees, and a bending angle θv of the extending portions 30S3 and 30S4 is set to an angle of approximately 90 degrees.

The image pickup apparatus 1C has the effects of the image pickup apparatuses 1, 1A and 1B, and further since heat transmission patterns 35C and 35CB are formed on the extending portions 30S3 and 30S4, respectively, the apparatus has a better heat radiation characteristic. In particular, since the wirings 33, etc. are not formed on the extending portions 30S3 and 30S4, heat transmission patterns having wider areas can be formed.

Besides, it is a matter of course that the same effects as the image pickup apparatus 1C can be obtained in an image pickup apparatus which is provided with a wiring board having three extending portions extending in directions orthogonal to each other.

### <Fifth Embodiment>

Next, an image pickup apparatus 1D according to a fifth embodiment will be described. The image pickup apparatus 1D is similar to the image pickup apparatuses 1, 1A, 1B and 1C, and therefore the same reference signs are assigned to the same elements and the description thereof is omitted.

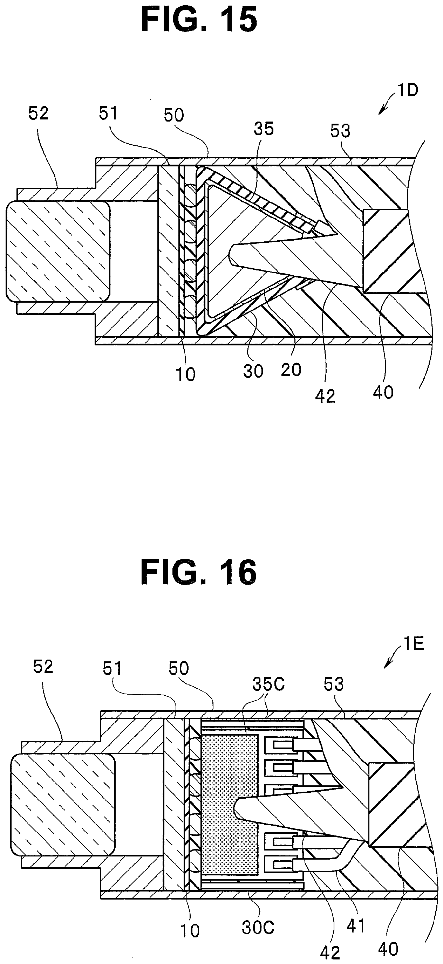

As shown in FIG. 15, the image pickup apparatus 1D further includes a cover glass 51 joined to the obverse surface 10SA of the image pickup device chip 10, an optical unit 52 constituted by a lens and a support body thereof, and an external cylinder portion 50 made of a metal. It is noted that FIG. 15 is a schematic view composed by sectional views along different planes so as to make the description easy, and the wirings 33, etc. are not shown. Further, it is usual that the optical unit 52 is constituted by optical members such as a plurality of lens, and a fixing member and a support body thereof, but the optical unit is schematically depicted.

A distal end portion of the cable 40, the image pickup device chip 10, the cover glass 51 and the wiring board 30 are housed in the external cylinder portion 50. That is, an inside dimension of the external cylinder portion 50 is substantially equal to the projected plane of the image pickup device chip 10. Inside the external cylinder portion 50, a non-conductive resin 53 having high thermal conductivity such as a silicone resin is filled.

Further, the image pickup apparatus 1D is provided with a block 20 which is a heat radiation member with which the extending portions are in contact. The block 20 is in contact with the second primary surface 30SB of the wiring board 30, and has a function as a fixing member for arranging the wiring board 30 in a predetermined space, i.e. in the interior 10S of the projected plane of the image pickup device chip 10. Further, the block 20 makes a joining work of the cable 40 to the wiring board 30 easy since the block 20 retains the wiring board 30 stably.

The block 20 also has a function as a reinforcing member for retaining the image pickup device chip 10 and the wiring board 30 to be integrated therewith and increasing mechanical strength. Specifically, there is a fear that the image pickup device chip 10 is deformed or broken by an external force since the image pickup device chip is formed of a silicone substrate, for example. However, the mechanical strength of the image pickup device chip 10 is increased by the block 20 being joined through the wiring board 30. Similarly, the strength of the wiring board 30 is increased as being joined to the block 20 although flexibility is substantially lost.

Further, the image pickup apparatus 1D is configured such that the wiring board 30 is always in a bent state at a predetermined angle without using a special jig or the like when bending the board since the wiring board 30 is fixed in a state of being in contact with the block 20. That is, the image pickup apparatus 1D is configured such that the wiring board 30, etc. are capable of being easily disposed in a predetermined narrow space.

It is noted that, in the present specification, "contact" includes not only a case of direct contact without any other member but also a case of being joined through a thin junction film. For example, a silicone resin having high heat conductivity can be used preferably as the junction film.

Further, the cable 40 of the image pickup apparatus 1D has a shield wire 42 which covers the plurality of lead wires 41 and is made of a mesh-like metal. The shield wire 42 is joined to the block 20 and the exterior cylinder portion 50. Therefore, heat of the block 20 and the exterior cylinder portion 50 is transmitted to the proximal end side efficiently through the shield wire 42 having high heat conductivity.

The image pickup apparatus 1D has the effects of the image pickup apparatuses 1-1C, and further is easy to manufacture and excellent in the heat radiation characteristic.

### <Sixth Embodiment>

Next, an image pickup apparatus 1E according to a sixth embodiment will be described. The image pickup apparatus 1E is similar to the image pickup apparatuses 1-1D and therefore the same reference signs are assigned to the same elements and the description thereof is omitted.

As shown in FIG. 16, the image pickup apparatus 1E has the wiring board 30C with the extending portions in four directions similarly to the image pickup apparatus 1C. Further, a part of the heat transmission pattern 35C is in contact with an inner surface of the exterior cylinder portion 50. It is noted that FIG. 16 is a schematic view similar to FIG. 15 and the wirings 33, etc. are not shown.

In the image pickup apparatus 1E, the heat generated by the image pickup unit 11 is transmitted to the exterior cylinder portion 50 efficiently through the heat transmission pattern 35C. Therefore, the image pickup apparatus 1E has the effects of the image pickup apparatuses 1-1D, and further is excellent in the heat radiation characteristic.

### <Seventh Embodiment>

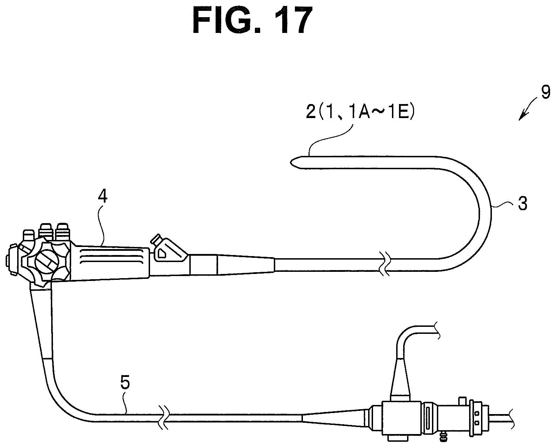

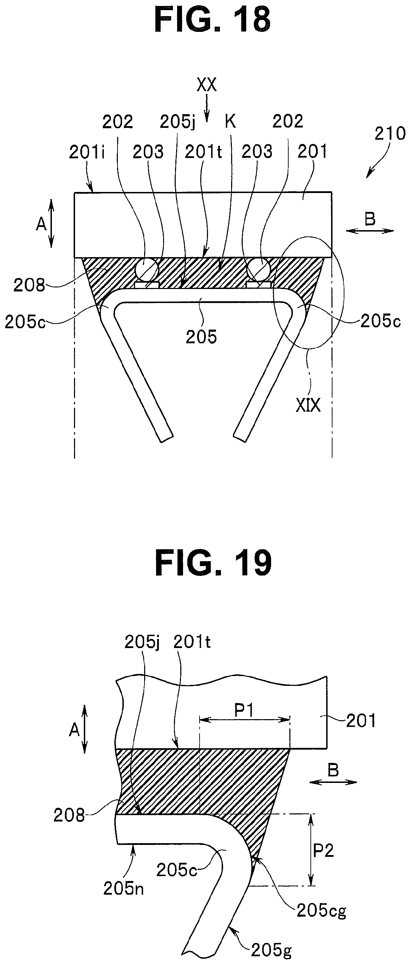

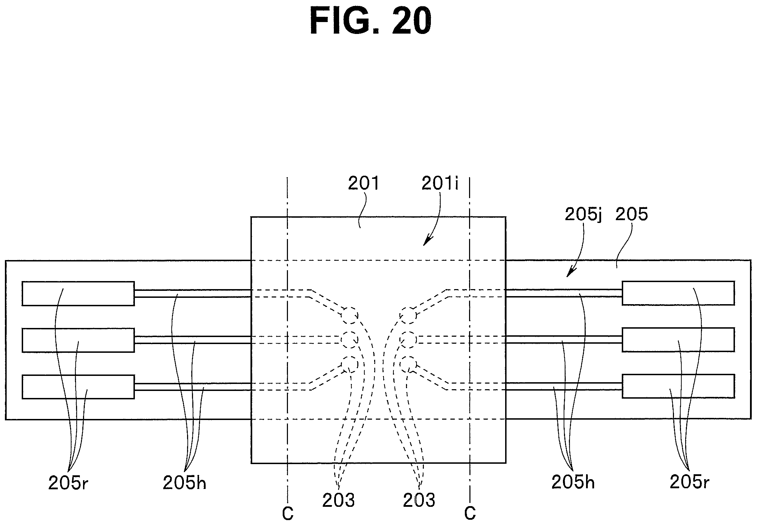

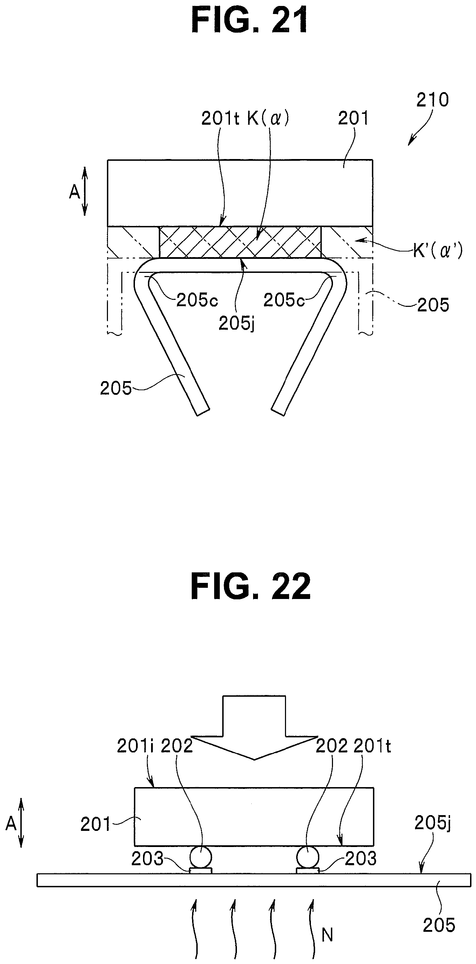

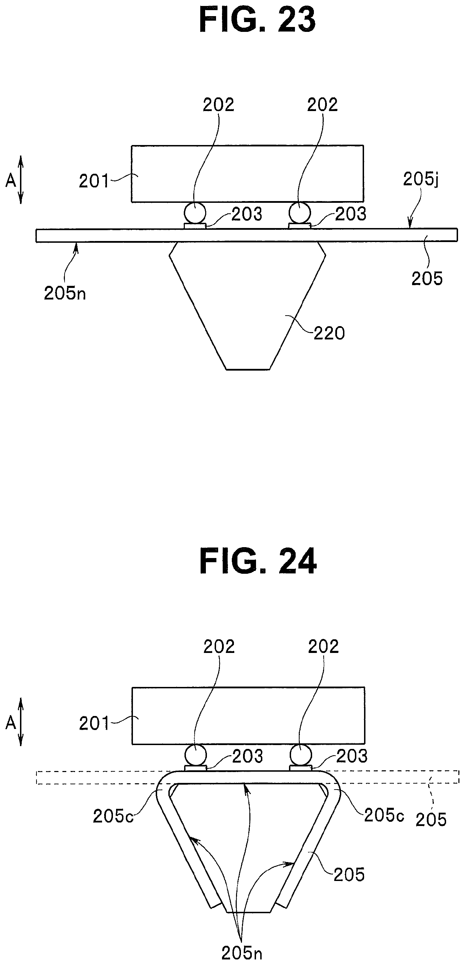

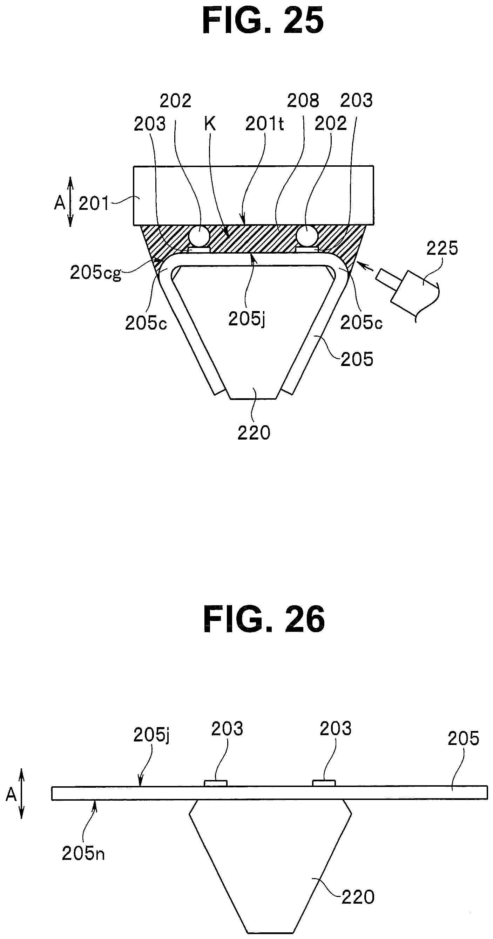

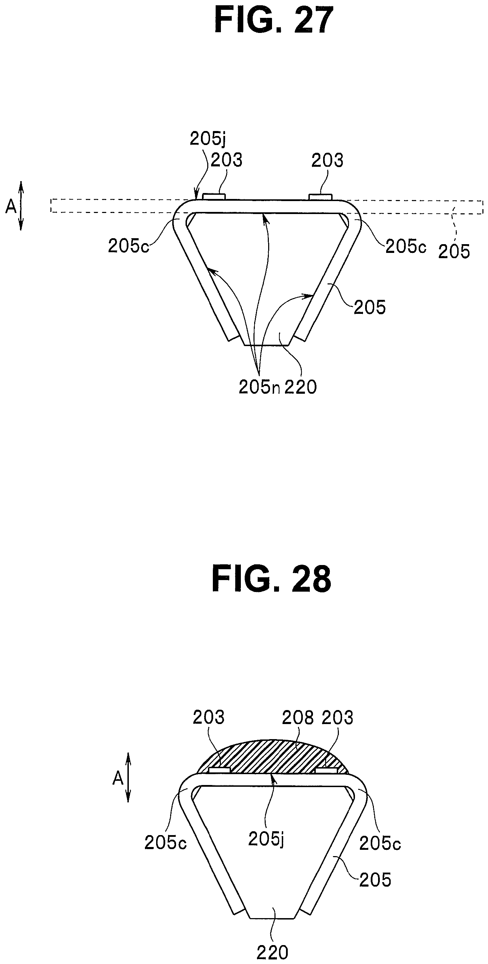

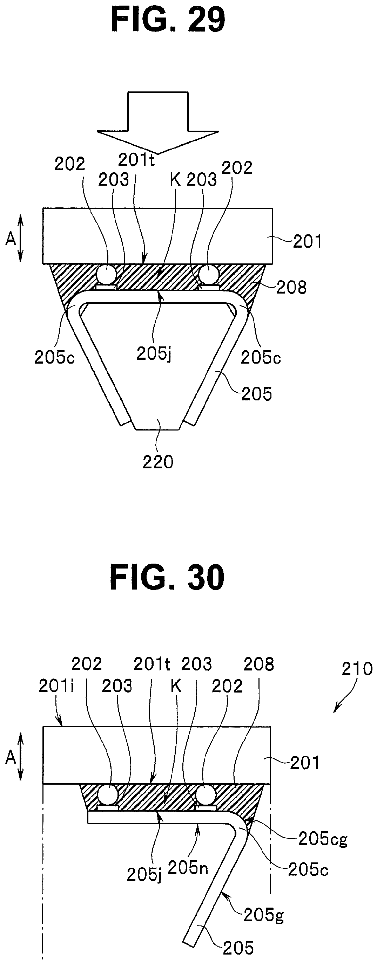

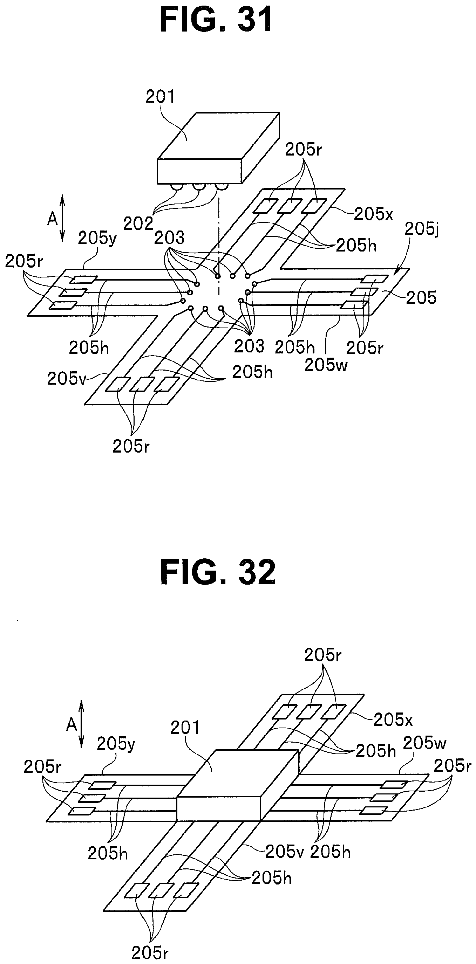

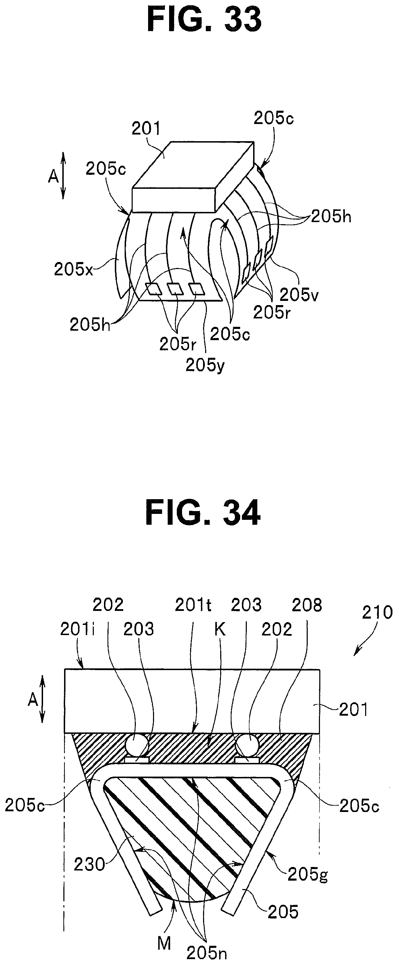

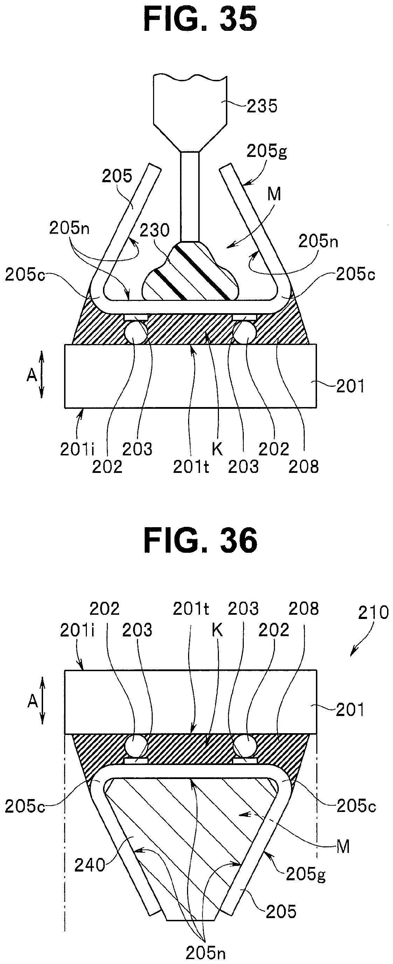

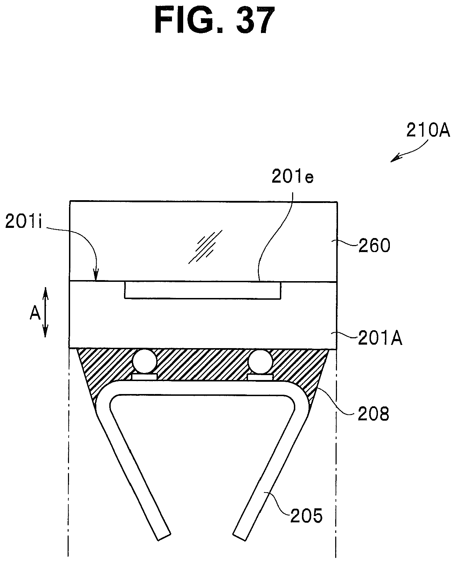

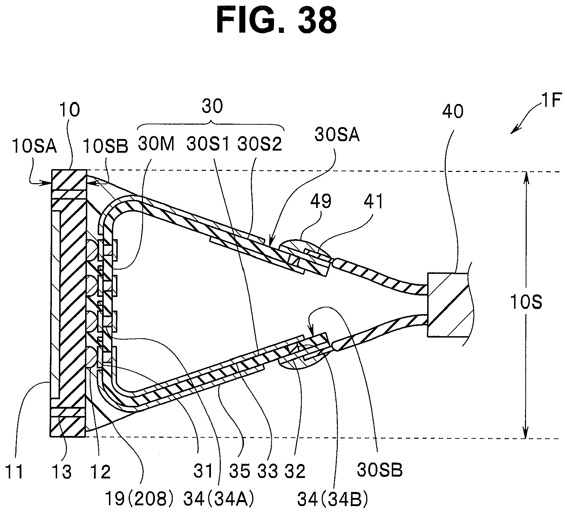

Next, an endoscope 9 according to a seventh embodiment will be described. As shown in FIG. 17, the endoscope 9 has the image pickup apparatus 1, 1A-1E at a distal end portion 2 of an insertion portion 3. Further, the endoscope 9 is provided with an operation portion 4 disposed at a proximal end side of the insertion portion 3 and a universal cord 5 extending form the operation portion 4.

At the operation portion 4, there are disposed various kinds of switches and the like to be operated by a surgeon while grasping the operation portion. The cable 40 of the image pickup apparatus 1 is inserted through the insertion portion and the universal cord 5 and connected with a main body unit (not shown), which performs image processing, etc., via a connector disposed at a proximal end portion of the universal cord.

Since the endoscope 9 is provided with the image pickup apparatus 1, 1A-1E, which is capable of being disposed in a narrow space and excellent in the heat radiation characteristic, at the distal end portion, the endoscope has the distal end portion with a small diameter and is excellent in thermal stability.

## Claims

1. An image pickup apparatus comprising:
an image pickup device chip (10) that has an image pickup unit (11) on an obverse surface (10SA) and junction terminals (12) on a reverse surface (10SB), the junction terminals being (12) connected with the image pickup unit (11) via through wirings (13);
a signal cable (40) having lead wires (41) connected with the image pickup unit (11); and
a wiring board (30) that is constituted by a central portion (30M) and a plurality of extending portions (30S1, 30S2, 30S3, 30S4) extending from the central portion (30M), and includes junction electrodes (31) formed at the central portion (30M) and joined to the junction terminals (12), terminal electrodes (32) formed at the extending portions (30S1, 30S2, 30S3, 30S4) and connected with the lead wires (41), wirings (33, 34) that connect the junction electrodes (31) and the terminal electrodes (32), and a heat transmission pattern (35, 35A, 35B, 35BB) formed in a region where the junction electrodes (31), the terminal electrodes (32) and the wirings (33) are not formed, the extending portions (30S1, 30S2, 30S3, 30S4) being bent and thereby the wiring board (30) being arranged within a projected plane of the image pickup device chip (10), **characterized in that**
the image pickup device chip (10) has a dummy junction terminal (12D) that has the same shape as the junction terminal (12S, 12G, 12V, 12P) and is not connected with the image pickup unit (11), on the reverse surface (10SB), and
the wiring board (30) has a dummy junction electrode (31D) that has the same shape as the junction electrode (31) and is connected with the dummy junction terminal (12D).

2. The image pickup apparatus according to claim 1, wherein the heat transmission pattern (35A) of the wiring board (30) is connected with the lead wire (41G) of the signal cable (40) at a ground potential.

3. The image pickup apparatus according to claim 2, wherein the wiring board (30) is a both-side wiring board with a first primary surface (30SA) and a second primary surface (30SB) each having a wiring layer, and the wiring board (30) has, on the first primary surface (30SA), a power wiring (33V) as a wiring for supplying power to the image pickup unit (11), a pulse wiring (33P) as a wiring for supplying a pulse signal to the image pickup unit (11), and the heat transmission pattern (35A), and has, on the second primary surface (30SB), a signal wiring (33) as a wiring for transmitting and receiving signals to and from the image pickup unit (11).

4. The image pickup apparatus according to claim 3, wherein the wiring board (30) has a second heat transmission pattern (35BB) on the second primary surface (30SB).

5. The image pickup apparatus according to claim 2, wherein the wiring board (30) has three or four extending portions (30S1, 30S2, 30S3, 30S4) extending from the central portion (3M) in directions orthogonal to each other, and the plurality of terminal electrodes (32) and the plurality of wirings (33) are formed on at least one of the extending portions (30S1, 30S2, 30S3, 30S4), and the heat transmission pattern (35C) is formed on at least one of the extending portions (30S1, 30S2, 30S3, 30S4).

6. The image pickup apparatus according to claim 2, comprising an external cylinder portion (50) made of a metal in which the image pickup device chip (10), the wiring board (30) and a part of the signal cable (40) are housed, and
a part of the heat transmission pattern (35C) is in contact with an inner surface of the external cylinder portion (50).

7. The image pickup apparatus according to claim 6, comprising a heat transmission member (35V) with which primary surfaces of the extending portions (30S1, 30S2, 30S3, 30S4) are in contact.

8. An endoscope comprising:
an insertion portion (3) having a distal end portion (2) at which the image pickup apparatus according to any one of claims 1 to 7 is disposed;
an operation portion (4) disposed on a proximal end side of the insertion portion (3); and
a universal cord (5) extending from the operation portion (4).

## Patentansprüche

1. Bildaufnahmevorrichtung, aufweisend:
einen Bildaufnahmegerät-Chip (10) mit einer Bildaufnahmeeinheit (11) auf einer Vorderseite (10SA) und Anschlussenden (12) auf einer Rückseite (10SB), wobei die Anschlussenden (12) mit der Bildaufnahmeeinheit (11) über Durchkontaktierungen (13) verbunden sind;
ein Signalkabel (40) mit Zuleitungsdrähten (41), die mit der Bildaufnahmeeinheit (11) verbunden sind; und
eine Leiterplatte (30), die aus einem Mittelteil (30M) und einer Vielzahl von sich von dem Mittelteil (30M) wegerstreckenden Abschnitten (30S1, 30S2, 30S3, 30S4) besteht, und umfasst: Anschlusselektroden (31), die an dem Mittelteil (30M) ausgebildet und mit den Anschlussenden (12) verbunden sind, Endelektroden (32), die an den sich wegerstreckenden Abschnitten (30S1, 30S2, 30S3, 30S4) ausgebildet und mit den Zuleitungsdrähten (41) verbunden sind, Verdrahtungen (33, 34), die die Anschlusselektroden (31) mit den Endelektroden (32) verbinden, und eine Wärmeabstrahlungsstruktur (35, 35A, 35B, 35BB), die in einem Bereich ausgebildet ist, in dem die Anschlusselektroden (31), die Endelektroden (32) und die Verdrahtungen (33) nicht ausgebildet sind, wobei die sich wegerstreckenden Abschnitte (30S1, 30S2, 30S3, 30S4) gebogen werden und dadurch die Leiterplatte (30) innerhalb einer projizierten Ebene des Bildaufnahmegerät-Chips (10) angeordnet ist, **dadurch gekennzeichnet, dass**
der Bildaufnahmegerät-Chip (10) auf der Rückseite (10SB) ein Blindanschlussende (12D) aufweist, das dieselbe Form wie das Anschlussende (12S, 12G, 12V, 12P) hat und nicht mit der Bildaufnahmeeinheit (11) verbunden ist, und
die Leiterplatte (30) eine Blindanschlusselektrode (31D) aufweist, die dieselbe Form wie die Anschlusselektrode (31) hat und mit dem Blindanschlussende (12D) verbunden ist.

2. Bildaufnahmevorrichtung nach Anspruch 1, wobei die Wärmeabstrahlungsstruktur (35A) der Leiterplatte (30) mit dem Zuleitungsdraht (41G) des Signalkabels (40) auf einem Massepotential verbunden ist.

3. Bildaufnahmevorrichtung nach Anspruch 2, wobei die Leiterplatte (30) eine beidseitige Leiterplatte ist, mit einer ersten Hauptoberfläche (30SA) und einer zweiten Hauptoberfläche (30SB), die jeweils eine Verdrahtungsschicht aufweisen, und wobei die Leiterplatte (30) auf der ersten Hauptoberfläche (30SA) aufweist: eine Stromverdrahtung (33V) als eine Verdrahtung, um die Bildaufnahmeeinheit (11) mit Strom zu versorgen, eine Impulsverdrahtung (33P) als eine Verdrahtung, um ein Impulssignal an die Bildaufnahmeeinheit (11) zu übertragen, und die Wärmeabstrahlungsstruktur (35A), und wobei die Leiterplatte (30) auf der zweiten Hauptoberfläche (30SB) eine Signalverdrahtung (33) aufweist, die als Verdrahtung zum Übertragen und Empfangen von Signalen an bzw. von der Bildaufnahmeeinheit (11) dient.

4. Bildaufnahmevorrichtung nach Anspruch 3, wobei die Leiterplatte (30) auf der zweiten Hauptoberfläche (30SB) eine zweite Wärmeabstrahlungsstruktur (35BB) aufweist.

5. Bildaufnahmevorrichtung nach Anspruch 2, wobei die Leiterplatte (30) drei oder vier sich wegerstreckende Abschnitte (30S1, 30S2, 30S3, 30S4) aufweist, die sich von dem Mittelteil (3M) in einem rechten Winkel zueinander erstrecken, und wobei die Mehrzahl von Endelektroden (32) und die Mehrzahl von Verdrahtungen (33) auf wenigstens einem der sich wegerstreckenden Abschnitte (30S1, 30S2, 30S3, 30S4) ausgebildet sind, und wobei die Wärmeabstrahlungsstruktur (35C) auf wenigstens einem der sich wegerstreckenden Abschnitte (30S1, 30S2, 30S3, 30S4) ausgebildet ist.

6. Bildaufnahmevorrichtung nach Anspruch 2, aufweisend einen äußeren Zylinderabschnitt (59) aus Metall, in dem der Bildaufnahmegerät-Chip (10), die Leiterplatte (30) und ein Teil des Signalkabels (40) untergebracht sind, und
wobei ein Teil der Wärmeabstrahlungsstruktur (35C) in Kontakt mit einer Innenoberfläche des äußeren Zylinderabschnitts (50) ist.

7. Bildaufnahmevorrichtung nach Anspruch 6, aufweisend ein Wärmeübertragungselement (35V), mit dem Hauptoberflächen der sich wegerstreckenden Abschnitte (30S1, 30S2, 30S3, 30S4) in Kontakt sind.

8. Endoskop, aufweisend:
ein Einführstück (3) mit einem distalen Endstück (2), an dem eine Bildaufnahmevorrichtung nach einem der Ansprüche 1 bis 7 angeordnet ist;
einem Bedienabschnitt (4), der an einem proximalen Ende des Einführstücks (3) angeordnet ist; und
einem Universalkabel (5), das sich von dem Bedienabschnitt (4) wegerstreckt.

## Revendications

1. Appareil de capture d'image comprenant :
une puce de dispositif de capture d'image (10) qui a une unité de capture d'image (11) sur une surface recto (10SA) et des bornes de jonction (12) sur une surface inverse (10SB), les bornes de jonction (12) étant raccordées avec l'unité de capture d'image (11) via des câblages traversants (13) ;
un câble de signal (40) ayant des fils conducteurs (41) raccordés avec l'unité de capture d'image (11) ; et
une carte de câblage (30) qui est constituée par une partie centrale (30M) et une pluralité de parties d'extension (30S1, 30S2, 30S3, 30S4) s'étendant à partir de la partie centrale (30M) et comprend des électrodes de jonction (31) formées au niveau de la partie centrale (30M) et assemblées aux bornes de jonction (12), les électrodes de borne (32) étant formées au niveau des parties d'extension (30S1, 30S2, 30S3, 30S4) et raccordées avec les fils conducteurs (41), les câblages (33, 34) qui raccordent les électrodes de jonction (31) et les électrodes de borne (32), et un motif de transmission de chaleur (35, 35A, 35B, 35BB) formé dans une région où les électrodes de jonction (31), les électrodes de borne (32) et les câblages (33) ne sont pas formés, les parties d'extension (30S1, 30S2, 30S3, 30S4) étant pliées et ainsi la carte de câblage (30) étant agencée dans un plan en saillie de la puce de dispositif de capture d'image (10), **caractérisé en ce que** :
la puce de dispositif de capture d'image (10) a une borne de jonction factice (12D) qui a la même forme que la borne de jonction (12S, 12G, 12V, 12P) et n'est pas raccordée avec l'unité de capture d'image (11), sur la surface inverse (10SB), et
la carte de câblage (30) a une électrode jonction factice (31D) qui a la même forme que l'électrode de jonction (31) et est raccordée avec la borne de jonction factice (12D).

2. Appareil de capture d'image selon la revendication 1, dans lequel le motif de transmission de chaleur (35A) de la carte de câblage (30) est raccordé au fil conducteur (41G) du câble de signal (40) à un potentiel de terre.

3. Appareil de capture d'image selon la revendication 2, dans lequel la carte de câblage (30) est une carte de câblage à deux côtés avec une première surface principale (30SA) et une seconde surface principale (30SB) ayant chacune une couche de câblage, et la carte de câblage (30) a, sur sa première surface principale (30SA), un câblage d'alimentation (33V) en tant que câblage pour fournir de l'énergie à l'unité de capture d'image (11), un câblage d'impulsion (33P) en tant que câblage pour fournir un signal pulsé à l'unité de capture d'image (11), et le motif de transmission de chaleur (35A), et a, sur la seconde surface principale (30SB), un câblage de signal (33) en tant que câblage pour transmettre et recevoir des signaux à et depuis l'unité de capture d'image (11).

4. Appareil de capture d'image selon la revendication 3, dans lequel la carte de câblage (30) a un second motif de transmission de chaleur (35BB) sur la seconde surface principale (30SB).

5. Appareil de capture d'image selon la revendication 2, dans lequel la carte de câblage (30) a trois ou quatre parties d'extension (30S1, 30S2, 30S3, 30S4) s'étendant à partir de la partie centrale (3M) dans des directions orthogonales entre elles, et la pluralité d'électrodes de borne (32) et la pluralité de câblages (33) sont formées sur au moins l'une des parties d'extension (30S1, 30S2, 30S3, 30S4), et le motif de transmission de chaleur (35C) est formé sur au moins l'une des parties d'extension (30S1, 30S2, 30S3, 30S4).

6. Appareil de capture d'image selon la revendication 2, comprenant une partie de cylindre externe (50) réalisée avec un métal, dans laquelle la puce de dispositif de capture d'image (10), la carte de câblage (30) et une partie du câble de signal (40) sont logées, et
une partie du motif de transmission de chaleur (35C) est en contact avec une surface interne de la partie de cylindre externe (50).

7. Appareil de capture d'image selon la revendication 6, comprenant un élément de transmission de chaleur (35V) avec lequel les surfaces principales des parties d'extension (30S1, 30S2, 30S3, 30S4) sont en contact.

8. Endoscope comprenant :
une partie d'insertion (3) ayant une partie d'extrémité distale (2) sur laquelle est disposé l'appareil de capture d'image selon l'une quelconque des revendications 1 à 7 ;
une partie de commande (4) disposée sur un côté d'extrémité proximale de la partie d'insertion (3) ; et
un cordon universel (5) s'étendant à partir de la partie de commande (4).
